Europäisches Patentamt

European Patent Office

Office européen des brevets

(11) Publication number: **0 220 702**
A2

# EUROPEAN PATENT APPLICATION

(21) Application number: 86114863.3

(22) Date of filing: 27.10.86

(51) Int. Cl.⁴: **C 12 N 5/00**, C 12 N 15/00, C 12 P 21/00, G 01 N 33/577, G 01 N 33/573

(30) Priority: 28.10.85 AU 3136/85

(43) Date of publication of application: 06.05.87
Bulletin 87/19

(84) Designated Contracting States: AT BE CH DE FR GB IT LI NL SE

(71) Applicant: **MABCO LIMITED C/- Douglas Heck & Burrell 3rd Floor, 370 Queen Street, Brisbane Queensland 4000 (AU)**

(72) Inventor: **Bundesen, Peter Gregory, 21 Tofanella Street Fig Tree Pocket, Brisbane Queensland (AU)**
Inventor: **Rylatt, Dennis Brian, 106 Howard Street Rosalie, Brisbane Queensland (AU)**

(74) Representative: **Hansen, Bernd, Dr.rer.nat. et al, Hoffmann, Eitle & Partner Patentanwälte Arabellastrasse 4, D-8000 München 81 (DE)**

(54) Method for the preparation of monoclonal antibody derived from immunoreactive trypsin.

(57)    A method for the preparation of monoclonal antibody (MAb) derived from immunoreactive tyrypsin (IRT) including the steps of:

(i) obtaining purified human IRT or extract containing same; and

(ii) forming antibody to said derivative or said extract by cloning antibody producing cells from an animal having been administered thereto said derivative of said extract.

There is also included a method of detection of IRT in a fluid sample using the aforementioned monoclonal antibodies wherein a fluid sample suspected of containing IRT is contacted with the aforementioned monoclonal antibodies and the resulting complex subjected to a detection step which may be RIA or EIA. There is also provided a method of diagnosis of cystic fibrosis depending upon the result of the protection step having regard to the concentration levels of IRT in the fluid sample. There is also provided a method of diagnosis of pancreatic damage wherein the presence of IRT in the fluid sample is analysed to determine the concentration levels.

2.

THIS INVENTION relates to the production of MAb derived from human immunoreactive trypsin (IRT) or derivatives thereof (eg. trypsin, trypsinogen and chymotrypsin, chymotrypsinogen) and the use of these MAb in the determination of the levels of IRT in blood, serum, lymph and faecal samples or any body fluid and in particular blood samples dried on filter paper.

Several authors have reported that the levels of IRT were elevated in new born children with cystic fibrosis (eg. King et al 1979 Lancet ii 1217-1219, Crossley et al 1979 Lancet 1, 472-474) and that these levels subsequently fell in most cases to values below normal. Consequently programs for the determination of the levels of IRT as a screening test have been initiated (Crossley et al (1981) Clin Chim Acta 113 111-121) as evidence has been accumulating in recent years that early institution of appropriate therapy significantly improves the prognosis (Oxenham et al 1977 Am. J. Dis. Child 131 973-975; Kraemer et al 1977 Helv. Paediats. Acta 32 107-114) avoiding perhaps years of inappropriate treatment and counselling.

Several assays have been developed (King et al above, Crossley et al above and Elias et al Lancet (1977) ii 1125) to screen for cystic fibrosis in neonates, which measure elevated levels of IRT in dried blood on paper samples. If high levels of IRT are found at 5 days of age the infants are retested at 1 month. If elevated levels persist, a positive diagnosis can be expected.

3.

The molecular species elevated in cystic fibrosis (CF) blood spots are believed to be the zymogens of trypsin (Crossley et al above, Geokas et al Amer. J. Physiol. 1979; 236; 77-83) but it is not clear in the various radioimmunoassay procedures whether the species being measured is the zymogen, the active enzyme or perhaps a combination of these antigens (O'Connor et al. Clin. Chim. Acta 1981; 114; 29-35; Iwaki et al Enzyme 1983; 29; 153-159). Obviously this could lead to discrepancies between the various assays which would depend on the major molecular species reacting with the particular antibody preparation used in each assay as well as the molecular species used as the trace antigen. These and other considerations have led to much debate on the value of the measurement of IRT and the role of such tests in any screening programme (Paediatrics 1983; 72,6 741-744). Other proteins that originate from the pancreas may also be released into the blood stream as a result of fibrocystic damage.

It also must be pointed out that the conventional assays referred to above are involving the use of polyclonal antisera and often require a large volume of sample to be analysed, are relatively complicated, require a high degree of technical skill and equipment and have not therefore proved particularly suitable for the large scale screening of neonates.

4.

It is therefore an object of the present invention to provide a diagnostic test for detection of CF in neonates by employing an improved assay procedure for detecting human IRT in blood which may be used on a clinical basis.

It is a further object of the invention to provide a method for the preparation of a monoclonal antibody (MAb) from IRT which may be used in the aforementioned clinical test.

Accordingly in one aspect the invention provides a method of preparation of the MAb including the steps of:

(i)     obtaining purified human IRT or extract containing same, and

(ii)    forming antibody to said derivative or said extract by cloning antibody producing cells from an animal having been administered thereto said derivative or said extract.

In step (i) a suitable antigenic extract could be obtained from human autopsy pancreas according to the method of Crossley et al Clin Chim Acta 1981, 111-121.

In step (ii) a suitable animal to which the derivative or extract thereof may be administered is a mouse or rat. A mouse is preferred. It is also preferred to administer the purified human IRT extract one or more times. This procedure is preferred so that the task of obtaining monoclonal antibodies specific to the derivative is simplified.

5.

After aministration the mice which have had derivative or extract administered thereto are suitably killed and the spleens removed for subsequent processing to form a cell suspension. Further purification of the cell suspension may take place (eg. by centrifugation) to isolate spleen white blood cells or leucocytes which may be fused with mouse myeloma cells.

The cloning technique may be broadly based on the technique described in Galfre et al Nature 266 550-2 (1977) where polyethylene glycol is used as the cell fusing agent to form a hybridoma cell which may then be cloned or recloned as desired suitably or the basis of limiting dilution using appropriate cell feeder layers.

Preferably for the cultivation of hybridoma cells well plates are utilized wherein cell suspensions are placed in each well with appropriate cell cultivation media.

It is preferred to remove samples of cells for screening assays and these may be carried out as described hereinafter. A number of the strongest growth wells are suitably chosen for maintenance on the basis of the screening assays. After the screening assays it is possible to choose a number of specific antibody producing clonotypes to produce monoclonal antibody secreting cell lines by limiting dilution.

On the basis of further screening assays carried out on samples taken from well plates incorporating the limiting dilution clonotypes a number of specific antibody produced clones may be chosen for expansion to mass culture.

6.

A suitable screening assay for use in the abovementioned process may comprise the steps of:

(a)     coating a surface with human IRT,

(b)     contacting the antigen in step (i) with monoclonal antibody derived from human IRT prepared as described above, and

(c)     subjecting the complex formed in step (ii) to a detection step.

Suitably in step (a) a well plate may be utilized in which human IRT was applied to the individual wells.

Subsequently monoclonal antibody derived from human IRT was then added to each well. An appropriate detection step which may be applied is an EIA step wherein an appropriate enzyme conjugate may be coupled to the complex and substrate subsequently added. Alternatively RIA, FIA, agglutination, adherance or chemiluminescence may be used as appropriate detection steps.

The purpose of the screening assay procedure referred to above is to ensure that the cells being tested are producing antibody specific to human IRT.

The invention also includes within its scope an assay procedure for detection of the presence of human IRT including the steps of:

(1)     contacting monoclonal antibody prepared from human IRT with a fluid sample suspected of containing the antigen, and

(2)     subjecting the complex formed in step (1) to a detection step.

7.

In the abovementioned assay the antigen is human IRT. The monoclonal antibody is prepared as described previously which is relevant to the particular human IRT antigen being assayed.

The detection step may be any one of those already described in relation to the screening assay procedure but is suitably ← RIA or EIA.

In regard to a suitable EIA procedure suitable enzymes that may be utilized include phosphatase peroxidase, urease or glucosidase. Suitably the sensitivity of the EIA may be increased by using appropriate amplification systems such as biotin-conjugated MAb with enzyme-conjugated avidin or streptavidin.

The presence of the human IRT may be used as a suitable diagnostic aid for cystic fibrosis as set forth in the experimental section hereinafter.

The fluid sample may be obtained from any suitable body fluid such as serum, plasma, blood, faeces or urine.

The assay procedure may be performed using a tube, bead, well plate or micro plate as described previously or may be carried out in any other suitable manner including conventional procedures. A "stick" procedure using a single elongate member may be utilized wherein monoclonal Ab is initially coated thereon before application of step (1) and (2).

In another embodiment of the invention the monoclonal antibodies can be covalently attached to

8.

beads of various sizes. Large beads (2-10 mm in diameter) are suitable for RIA, EIA, FIA tests while small beads (0.1-2.0 μ in diameter) enable a quick (eg. 2-3 minute) test to be carried out for the presence of antigen in serum plasma or other body fluids. The beads may be formed from polystyrene, nylon, glass or other suitable material. In relation to polystyrene, the MAb can be coupled thereto using the carbodiimide method as described in Molday et al J. Cell Bio 64 75 (1975). For nylon beads a suitable coupling procedure is described in Hendry and Herrman J. Immun. Method 35 285 (1980) using glutaraldehyde. For glass beads a suitable coupling procedure using silaning agents may be utilized as described in U.S. Patent 4210723. In these bead assays or latex assays when the beads which have already been coupled to MAbs are tested with test serum or plasma or other body fluid they must be checked for agglutination by use of a suitable calibration standard. In this embodiment latex particles or beads are prepared as uniform spheres of known refractive index and used as calibration standards for light scattering magnification, shadow angle, thickness of shadow material, scanning and transmission electron microscopy or laser scattering.

When capture-tag experiments were carried out as discussed hereinafter an antihuman IRT capture MAb was bound to a support surface and was tested with serum or other body fluid suspected of containing human IRT. When tagged with a second suitably labelled MAb which bound to the captured

9.

human IRT antigen the subsequent detection step provided a precise assay for the presence of human IRT in the sample.

In the following Experiments human IRT was prepared as described above in the Crossley reference. Alternatively, commercially prepared material may be used (Calbiochem. Catalogue No. 650257).

When capture tag experiments were carried out as discussed hereinafter the MAb of interest are subjected to a two-site assay procedure involving the formation of a ternary complex comprising first MAb, antigen and labelled second MAb. The antigen is contained in the fluid sample being tested and it was noted that no MAb could act as both tag and capture MAb thus indicating that the antigen had a single reactive site or epitope. The two site assay can be carried out as described previously using any suitable support surface such as a tube, multiwell plate dipstick, plastics bead or latex beads. The assay may be of the "forward", simultaneous or "reverse" types as described hereinafter.

The "forward" type assay involves the capture MAb being bound to the support before being contacted with the fluid sample so as to form a binary complex of antigen-capture MAb before contacting the binary complex with labelled tag MAb. This may be varied to form a simultaneous assay wherein fluid sample capture MAb bound to the support surface and labelled tag MAb may all be contacted simultaneously to form the ternary complex.

10.

However, a "reverse" assay technique may be utilized wherein labelled tag MAb is added to the fluid sample to form a binary complex which is subsequently contacted with the capture MAb bound to the support surface so as to form the ternary complex.

It further will be appreciated that in the amplified assay systems described above a quaternary complex could be formed comprising capture MAb, antigen, biotin-conjugated tag MAb and enzyme conjugated avidin or streptavidin.

It will also be appreciated that in some cases a precoated support surface can be utilized wherein capture MAb is covalently bound thereto by any suitable method eg. by the aforementioned carbodiimide method.

In a further embodiment the assay procedure of the invention may be used for diagnosis of pancreatic damage and/or pancreatitis in humans. In this context it will be appreciated that detection of increased levels of IRT in human plasma or serum is indicative of pancreatic damage. Alternatively abnormally low levels of IRT in human plasma or serum can also be indicative of exocrine pancreatic insufficiency.

EXPERIMENTAL

Cell Fusion and Selection of Hybrids

Spleens were removed aseptically from 5 immunized mice killed by cervical dislocation three days after an injection of human IRT. Previously the mice had been

11.

immunized with two injections of human IRT prepared as reported in the aforementioned Crossley reference. The spleens were placed in two 60 mm petri dish (Falcon, 3001, Oxnard, Calif.) containing 5 ml complete medium (85% RPMI 1640, 15% foetal calf serum, 100 I.U.ml penicillin, 100 µg/ml streptomycin and $2 \times 10^{-1}$ M. Glutamino; Gibco, Grand Island, N.Y.). A cell suspension was prepared by decapsulating the spleens with 2X18 gauge needles attached to 3 ml disposable syringes with the last cm of the tip bent through an angle of 60 degrees. The cell suspensions were then aspirated into a 10 ml syringe fitted with a 22 gauge needle and ejected with moderate pressure. This operation was performed twice before filtering the cells into a Falcon 2001 tube through a fine mesh stainless steel screen to remove larger cell clumps and debris.

The cell suspensions were allowed to stand for 5 minutes at room temperature to allow smaller clumps and membrane fragments to settle before transferring the cell suspensions to a fresh Falcon 2001 tubes. The cells were centrifuged at 350G for 5 minutes at room temperature and the supernatants were decanted from the first cell pellet to a fresh tube and spun at 700G for five minutes to give a second cell pellet and the two pellets were pooled and resuspended in 5 ml complete medium. The spleen white blood cells (SWBC) were then counted and their viability estimated by Turks and Trypan blue stains respectively, and $100 \times 10^{6}$ viable SWBC were placed in separate Falcon 2001 tubes in a total volume of 5 ml complete medium. The NS-1 myeloma

12.

cells to be used for fusion, were washed once by centrifugation at 380G for 15 minutes at room temperature and adjusted to $5 \times 10^6$ viable cells/ml in complete medium.

Twenty-five $\times 10^6$ NS-1 and $100 \times 10^6$ immune SWBC were mixed and spun at 350G for 5 minutes at room temperature. The supernatant was decanted, the remaining medium was carefully removed with a Pasteur pipette and 3 ml of a 42% (w/v) solution of polyethylene glycol (PEG. MW1540) (Baker Chemical Co., New Jersey) in RPMI 1640 containing 15% (v/v) dimethyl sulfoxide (DMSO) at 37 degrees C was added with a 5 ml glass disposable pipette (Corning Glass, Corning, N.Y.) and the cells were resuspended with the same 5 ml pipette for 30 seconds with the aid of an electric pipetter (Pipet-aid Drummond Scientific Co., Broomall, Pa.). The PEG-cell suspension was allowed to stand for a further 30 seconds at room temperature before adding 5 ml complete medium, dropwise, with a Pasteur pipette, over a period of 90 seconds with constant flicking of the tube, sufficient to ensure complete mixing with the viscous PEG solution. A further 5 ml complete medium was immediately added and mixed by inversion and the cell suspension was allowed to stand for a further 150 seconds at room temperature before centrifugation at 350G for 5 minutes at room temperature. The supernatant was decanted and the cell pellet was gently resuspended in 5 ml complete medium using a 5 ml pipette with the electric pipetter; extreme care was taken not to break up all cell clumps. Using a Tridak stepper (Bellco Glass Inc., Vineland, N.J.), 0.05 ml of the cell suspension

0220702

13.

was added to each well of 4 Costar 24 well plates (Costar 3524, Cambridge, Mass.) containing $1 \times 10^6$ normal BALB/c mouse SWBC as feeder cells in 1 ml complete medium containing $10^{-4}$ M Hypoxanthine (Sigma), $4 \times 10^{-7}$ M Aminopterin (Sigma), $1.6 \times 10^{-5}$ M Thymidine (Sigma) and $4 \times 10^{-8}$ M 2-Mercaptoethanol (HAT medium) hereinafter referred to as $1^0$ fusion plates. In all six separate fusions were performed using this methodology.

The $1^0$ fusion plates were then placed in a humidified 5% $CO_2$ 95% air atmosphere at 37 degrees C. The cells were first fed either on days 5 or 7 and thereafter when necessary, with 0.5 ml fresh HAT medium. Generally, on day 10, 0.5 ml of the medium was removed for the screening assay from each well showing hybridoma growth and 0.5 ml fresh HAT medium was replaced. A number of the strongest growth wells were chosen for maintenance on the basis of the screening assay. The chosen wells were allowed to grow to confluency in the original well ($1^0$ well), then each was split in half and transferred to a fresh well ($2^0$ well) of a 24 well Costar plate ($2^0$ plate). The wells were checked daily and expanded to a 2nd, 3rd or 4th well of the $2^0$ 24-well Costar plate when necessary. From days 14-28, cells were fed with HT medium. When there was strong growth in at least 2 wells of the $2^0$ plate, supernatant from one well of each clonotype was chosen for rescreening and a number of specific antibody producing clonotypes were chosen from the results of the second screening assay to produce monoclonal antibody secreting cell lines by limiting dilution.

14.

## Cloning of Hybridomas

One 2° well of each chosen clonotype was resuspended and the number of viable cells per well was estimated by Trypan blue exclusion. Immediately before plating each clonotype, the relevant series of dilutions were made in HT medium or complete medium (if the cells were older than 28 days post fusion) to give a frequency of 0.5 cells/0.05 ml. This volume was then added with a Tridak stepper to each well of a 96 well flat bottomed tissue culture plate (Flow Laboratories, Mississauga, Ontario, Canada) (L D plate) containing 1X10$^5$ normal mouse spleen feeder cells in 0.1 ml HT or complete medium. The LD plates were then placed in a 37 degree C humidified 5% CO$_2$, 95% air atmosphere and screened for clonal growth 7-10 days later. From each positive growth well, 0.1 ml supernatant was removed for screening and these wells were fed for the first time with 0.1-0.15 ml HT or complete medium. On the basis of the LD screening assay, a minimum of 2 of the "better" specific antibody-producing clones were finally selected for expansion to mass culture.

Alternatively if it was desired to obtain a large amount of MAb, female BALB/c mice were given an intraperitoneal injection of 0.5 ml 2, 5, 10, 14 tetramethyl-pentadecane (Pristane, Aldrich Chemical Corp., Milwaukee, Wisconsin) 14 days prior to the injection of 2x10$^6$ viable hybridoma cells and ascites fluids were collected from the mice 12 to 14 days after injection of the

15.

cells. The ascitic fluid was clarified by centrifugation and MAb were recovered by precipitation with 45% ammonium sulphate and stored at either 4 degrees C or -70 degrees C in phosphate buffered saline (PBS) containing 0.01% sodium azide.

## Monoclonal antibody screening assay

The wells of a 96 well U bottomed microtest plate (Disposable Products Pty. Ltd., Adelaide, South Australia) were coated by adding 50 µl of human IRT (10 µg/ml) in PBS for 1 hr at room temperature (25 degrees C). Excess antigen was removed by inverting and tapping the plate and the plate was then washed three times with PBS containing 0.05% Tween 20 (Sigma Chemical Corp., St. Louis, Missouri). Clones secreting MAb to human IRT were then detected by adding 50 µl of tissue culture supernatant to each well and incubating for 1 hr at room temperature. Unbound MAb was removed by inversion and tapping and the plate was washed three times with PBS/Tween. One hundred µl of a 1/1000 dilution of peroxidase conjugated rabbit anti-mouse immunoglobulin (Dakopatts, Copenhagen, Denmark) in PBS/Tween was added and allowed to incubate a further one hour at room temperature. The plate was again inverted and washed three times with PBS/Tween and 100 µl of activated substrate (immediately before use 10 µl of a 3% solution of hydrogen peroxide was added to 10 ml of a substrate solution containing 50 mM citrate, 2.5 mM of 0-tolidine dihydrochloride (0-tolidine, Sigma Chemical Co. recrystallized from dilute HCl) 0.035 mM

16.

EDTA pH 4.5) was added to each well. The colour reaction was stopped after 10 minutes by the addition of 50 µl of 3M HCl which caused a colour change from blue to yellow and the absorbance was recorded at 450 nm on a Titertek multiskan.

Iodination of MAb:

MAb were iodinated after the method of Fraker and Speck described in Biochem. Biophys. Res. Commun. **80** 849-857. Iodogen (Pierce and Warriner, Cheshire, U.K) was dissolved in dichloromethane (0.2µg/ml) 15µl of this solution was added to a test tube and a thin film of iodogen was deposited on the bottom of the tube after evaporation of the dichloromethane with gentle warming. The tube was then placed in an ice bath and 100 µg of MAb in 0.2 PBS and 0.1 mCi Na$^{125}$I added and the tube agitated vigorously for 15 min. The reaction mixture was then dialyzed against PBS to remove unincorporated Iodine and the Na$^{125}$I MAb was precipitated with 45% $(NH_4)_2SO_4$ and finally redissolved in PBS containing 0.01% sodium azide. Alternatively, iodinations were carried out using the Chloramine T method (6) described in Biochem J. **89** 114.123 except that only a 2 M excess of Chloramine T was used.

Peroxidase conjugation

Conjugation of the IRT monoclonal antibodies was carried out by a modification of the method of Nakane and Kaiwoi, J. of Histochem and Cytochem **22** 1084-91, (1974) with periodate oxidized peroxidase. 5 mg/ml peroxidase in distilled water was mixed with a 1/5 volume of 0.1M sodium

17.

periodate for 20 minutes at room temperature and unreacted periodate was removed by gel filtration on a column of Sephadex G25 equilibrated with 0.001M citrate pH 4.5. Monoclonal antibody (in PBS) was added in a ratio of 2 mg antibody per mg peroxidase and the pH was immediately adjusted to pH 9.0 - 9.5 by the addition of 1M sodium carbonate, pH 9.5. The reaction was allowed to proceed for 2-3 hours at room temperature with occasional mixing and stopped by the addition of 1/10th volume 2.0M ethanolamine pH 9.5 Barbour, H.M. J of Immunol Meth. 11, 15-23, (1976). After sitting overnight at 4 °C, ethanolamine was removed by gel filtration on a Sephadex G25 column equilibrated with PBS and the enzyme conjugate was stored at 4° C in the presence of 0.01% methiolate.

Biotin Labelling of IRT-4B2/41

This procedure included the following steps:

(i)     1.0 ml MAb (3 mg/ml) dialysed against 0.1 M Na HCO₃ for 3 hours at 4° C,

(ii)    0.1M N-Hydroxy succinimido biotin (BNHS) (obtained from Sigma-catalogue No. H-1759) in dimethyl sulfoxide (34.1 mg/ml) was prepared and 60 μl was added per ml of MAb added,

(iii)   incubation at room temperature for 1 hour using a mixing wheel,

(iv)    subsequent dialysis against PBS containing 0.1% sodium azide at 4° C for 24 hours and

(v)     storage at 4° C until use.

18.

## Protein Determination

Protein determination was carried out by the method of Rylatt and Parish Analytical Biochem, 121 213-214 (1982).

## Capture/Tag Experiments

Antigen capture/tag experiments were performed by incubating each well of a PVC 96 well microtitre plate with 50 µl (10 µg/ml) of each of the relevant MAb in PBS for 1 hour at room temperature. Unbound MAb was removed by inversion and tapping the plate followed by washing with PBS/Tween as described for the screening assay. Antigen capture was then achieved by adding 50 µl of antigen (0-10 µg/ml) in PBS/Tween with 5-µg/ml Aprotinin to the MAb coated wells for 1 hour at room temperature. The wells were washed as previously described. Capture antigen was then tagged with $^{125}$I conjugated MAb by adding 100,000 cpm in 50µl (20 µg/ml) of the various $^{125}$I conjugated MAb in PBS/Tween to each well for one hour at room temperature. After washing, the presence of bound conjugate was determined by counting in a Kontron gamma counter.

Alternatively antigen capture/tag experiments were performed by incubating each well of a 96 well microtitre plate with 50 µl (10 µg/ml) of each of the relevant MAb in PBS for 1 hour at room temperature. Unbound MAb was removed by inversion and tapping the plate followed by washing with PBS/Tween as described for the screening assay. Antigen capture was then achieved by adding 50 µl of each antigen (0-1 mg/ml) in PBS/Tween to the MAb coated wells for 1 hour

19.

at room temperature. The wells were washed as previously described. Captured antigen was then tagged with peroxidase conjugated MAb by adding 50 μl (1 μg/ml) of the various peroxidase conjugated MAb in PBS/Tween to each well for one hour at room temperature. After washing, the presence of bound conjugate was determined by the addition of 100 μl substrate as described in the screening assay.

20.

RESULTS

Specificity

Several hundred hybridoma clones secreting MAb against human IRT were initially identified by enzyme iummunoassay and a number were chosen for subcloning and ascitic fluid production followed by the development of Capture/Tag assays.

Capture/Tag antihuman IRT MAb combinations

Four Capture/Tag combinations were developed from the MAb raised against human Trypsin as previously described, and are as follows:

(1) Capture MAb IRT-3B6/140-157 with Tag MAb IRT-2D1/182

(2) Capture MAb IRT-3B6/140-157 with TAG MAb IRT 3D6/84

(3) Capture MAb IRT-3D6/84 with Tag MAb IRT-2D1/182

(4) Capture MAb IRT-3C4/54 with Tag MAb IRT-4B2/41

The results obtained with these 4 Capture/Tag MAb combinations with varying concentrations of human IRT are shown in the following table.

| IRT ng/ml | Assay 1 Radiometric IRT 3B6/140-157 2D1/82 cpm(I-125) | Assay 2 Radiometric IRT 3B6/140-157 3D6/84 cpm(I-125 | Assay 3 Radiometric IRT 3D6/84 2D1/182 cpm(I-125) | Assay 4 EIA IRT 3C4/54 4B2/41 A420nm |
|---|---|---|---|---|
| 1000 | 12123 | 4462 | 10011 | 2.0 |
| 100 | 4728 | 2207 | 5581 | 1.2 |
| 10 | 1035 | 573 | 719 | 0.7 |
| 1 | 359 | 327 | 303 | 0.2 |
| 0 | 234 | 293 | 191 | 0.1 |

21.

## Radiometric Assay for human IRT in biological samples (IRT-3B6/140-157; IRT-2D1/182)

As previously described suitable samples obtained from neonates may contain blood, plasma or serum or alternatively as suited for a neonatal screening programme for Cystic Fibrosis may be in the form of dried blood spots, plasma spots or serum spots on suitably absorbant material which is generally, though not restricted to, paper.

### Assay Method

Three millimetres whole blood spots punched from screening cards were incubated overnight at room temperature in tubes containing 300μl of phosphate buffered saline pH 7.4 containing 0.05% Tween 20 in the presence of 6.4 mm polystyrene beads coated with IRT-3B6/140-157. The following morning, 100μl of I-125 labelled MAb IRT-2D1/182 was added to each tube and incubated for a further hour at room temperature and after washing the radioactivity remaining bound to the beads was determined and the results are shown in the following diagrams (a & b).

(a) Retrospective Study (IRT-3B6/140-157; IRT-2D1/182)

IRT concentrations determined retrospectively showing confirmed CF (● ) and the range of grouped control samples (I————I) from 5 day blood spots stored for varying periods of time.

Blood samples collected at five days of age from 39 confirmed cases of CF which had been stored at room temperature on screening sample cards for up to 3 years were assayed. These samples were tested against control samples matched for date and location of birth. The assay distinguished elevated IRT levels in 36 of the 39 CF infants samples compared to the matched controls. Two of the samples did not show elevated levels possibly because of sample denaturation and the remaining CF sample also failed to show any detectable IRT by the Crossley radio-immunoassay (when originally tested). This particular infant required extensive pancreatic enzyme supplement.

(b)   Prospective Study (IRT-3B6/140-157; IRT-2D1/182)

Frequency  distribution of IRT concentrations from 5 day blood spots.

Samples  collected  at  5 days of age from 5485  infants  were tested and 27 infants  were  detected with elevated IRT levels ( > 125ng/ml).  On retesting these 27 infants at 1 month of age (resample  rate  0.5%) only 2 infants had elevated IRT  levels and were subsequently confirmed as cystic fibrotic.

Comparison of the IRT 3B6/140-157 and IRT 2D1/182 Radiometric and EIA Systems

To detect IRT from 3mm blood spots an alternative to the I-125 radiometric assay is the use of an enzyme conjugated tag MAb IRT 2D1/182. The assays are performed identically, with the exception that the captured antigen is tagged by the addition of 100 µl of peroxidase conjugated IRT 2D1/182 in PBS Tween to each test and incubated for 1 hour at room temperature. After washing, the presence of bound conjugate is determined by the addition of substrate as described for the screening assay. The results for both assays on a set of blood samples are shown below:

| Sample No | Radiometric Assay ng/ml | EIA ng/ml |
|-----------|-------------------------|-----------|
| 150009 | 184 | 140 |
| 151556 | 30 | 38 |
| 153673 | 324 | 363 |
| 156200 | 93 | 54 |
| 158820 | 218 | 203 |
| 159515 | 194 | 94 |
| 162845 | 185 | 85 |
| 167917 | 156 | 62 |
| 169765 | 500 | 496 |
| 167296 | 404 | 409 |
| 171188 | 99 | 180 |
| 171374 | 71 | 83 |
| 177331 | 59 | 79 |
| 178075 | 82 | 48 |
| 191228 | 50 | 45 |
| 193672 | 249 | 183 |
| 199138 | 332 | 331 |
| 203753 | 161 | 111 |

## An Alternative IRT EIA Screening System (IRT-3C4/54; IRT-4B2/41)

An alternative 96 well plate amplified enzyme immunoassay was developed to increase both sensitivity and ease of use. As previously described, the assay is performed with 3mm whole blood spots punched from screening cards. The spots are incubated for between 2 - 24 hours at room temperature in 100 µl of biotin labelled MAb IRT-4B2/41 diluted in phosphate buffered saline pH 7.4 containing 0.05% Tween 20 in the wells of a 96 well microtitre plate coated with IRT-3C4/54. After incubation, the plates are washed as described in the screening assay and 50 µl of HRP labelled streptavidin (Amersham, catalogue No RPN 1231) is added to each well and incubated for 15 minutes at room temperature. After washing the plates as described for the screening assay, substrate is added to each well to react with the bound enzyme for 20 minutes. The absorbance of standards and unknowns are then read at 410nm in a microtitre plate reader.

The range of the assay detection was determined by serial dilution of standard containing a preassayed concentration of IRT. The minimum detectable plasma IRT concentration which could be distinguished from zero in a bloodspot was 1.5ng/ml. The binding increased continually with trypsinogen concentration up to 2500ng/ml. The performance of this assay is described in the retrospective study (figure a) and prospective study (figure b).

<u>Figure a</u>   IRT concentrations determined retrospectively   using IRT-3C4/54   and IRT-4B2/41 showing confirmed CF   ( ● ) and the range   of grouped control samples (I———I) from 5   day   blood spots stored for varying periods of time.

The   results of this retrospective study (figure a)   utilising and   enzyme   immunoassay   are equivalent to   those   using   the combination   IRT-3B6/140-157 and IRT-2D1/182 in an I-125 assay show that the assay can distinguish elevated IRT levels in   36 of   the   39   CF   infants'   samples   compared   to   the   matched controls.

<u>Prospective Study (IRT-3C4/54; IRT-4B2/41)</u>

Figure b:

Frequency distribution of IRT concentrations from 5 day blood spots.

Blood spot samples collected from all infants (N=16500) for routine newborn screening over a six month period were assayed. Any sample with an IRT concentration greater than 140ng/ml plasma was reassayed and if this replicate assay value was again above the cut off, a second sample, to be collected at one month of age, was requested. Replicate assays were performed on 160 five day samples and of these 96 infant samples had a mean level greater than 140ng/ml (resample rate, 0.6%). On receipt of the second sample from these 96 infants, the assay was again performed. Any infant with an IRT level greater than 140ng/ml was referred for sweat test and clinical examination. In 7 infants the level was persistently elevated

chloride analysis.

## Assay Advantages (IRT-3C4/54; IRT-4B2/41)

This assay offers several advantages over exisitng methods used in neonatal screening for cystic fibrosis. The assay employs the specificity available from the use of monoclonal antibodies and demonstrates the sensitivity available from an amplified immunoassay two site procedure. This sensitivity may also be useful for monitoring the deterioration of the cystic fibrosis pancreas as it approaches a non-secreting state. A particular advantage of this assay format is that the label is introduced into one of the MAb and not the antigen. Consequently there is very little likelihood of damage to trypsinogen which can occur during the preparation of tracer for the conventional competition radioimmunoassay. In addition, the use of a solid phase support eliminates the need for addition of precipitating antibodies and polyethyleneglycol. Centrifugation and aspiration are not required, thus reducing the labour and risk of error involved in handling large numbers of test tubes.

## Tests for Pancreatic Function

Serum or plasma IRT determination has been recommended as a specific test for pancreatic diseases (Temler RS and Felber JP: Radioimmunoassay of human plasma trypsin. Biochem. biophys. Acta 445: 720-728. 1976; Elias E, Redshaw M circulating concentrations of immunoreactive trypsin. Lancet i: 66-68, 1977)

As a further embodiment of this invention these IRT MAb in the appropriate assay format may be used to measure concentrations of IRT in patient plasma or serum to aid the diagnosis of pancreatic diseases.

As previously described, suitable samples obtained from patients may be blood, plasma or serum.

Assay Method

(1)   50 μl of capture MAb (IRT 3B6/140-157 or IRT-3C4/54) (10 μg/ml) in PBS is incubated in each well of a 96 well microtitre plate for one hour at room temperature. Unbound MAb is removed by inversion and tapping the plate followed by washing as described for the screening assay. Alternatively a pre-coated plate may be used.

(2)   IRT antigen standards or test samples appropriately diluted in PBS/T are added to the plate and antigen capture is allowed to proceed for one hour at room temperature. The wells are washed as previously described.

(3)   Captured IRT antigen is then tagged by adding horseradish peroxidase conjugated with MAb in PBS/T to each well for one hour at room temperature. (As previously described, HRP conjugated IRT-2D1/152 is used with capture IRT-3B6/140-157 and HRP conjugated IRT-4B2/41 is used with .pa capture IRT-3C4/54) and the wells are washed.

(4)    One  hundred  microlitres  of $H_2O_2$  (0.003%  $H_2O_2$ ) activated ABTS substrate (2.2' Azinodi-(3-Ethylbenzthiazolone sulphonic acid) Sigma catalogue No A-1838) is added to each well  and incubated for 20 minutes at  room  temperature:  The reaction is stopped with 0.05ml 1% oxalic acid.

(5)    The plates are read at 420nm on a microtitre  plate reader  and IRT concentrations of test samples are  determined for the appropriate standard curve.

The results are shown in the following table:

### IRT-3C4/54 with IRT-4B2/41

| Standard Curve | | Test Samples | IRT-ng/ml |
|---|---|---|---|
| IRT-ng/ml | A420nm | | |
| 0 | - | Normal Plasma | 47 |
| 8 | 0.249 | " | 63 |
| 16 | 0.459 | " | 46 |
| 32 | 0.854 | " | 42 |
| 63 | 1.698 | confirmed pancreatitis plasma | 169 |
| 125 | 1.942 | " | >500 |
| 250 | 2.465 | " | 150 |
| 500 | 2.585 | " | 110 |
| | | " | 100 |

### IRT-3B6/140-157 with IRT-2D1/152

| Standard Curve | | Test Samples | IRT-ng/ml |
|---|---|---|---|
| ng/ml | A420nm | | |
| 0 | 0.- | Normal Plasma | 1120 |
| 47 | 0.041 | " | 1452 |
| 94 | 0.101 | " | 994 |
| 188 | 0.157 | " | 659 |
| 375 | 0.323 | confirmed pancreatic plasma | 1785 |
| 750 | 0.586 | " | 2523 |
| 1500 | 1.007 | " | >3000 |
| 3000 | 1.353 | " | >3000 |
| | | " | >3000 |

Both assay formats clearly distinguished plasma from patients with confirmed pancreatic disease from normal plasma values.

Assay Advantages

IRT is an ideal marker for disease or damage of the exocrine pancreas as the endopeptidases are unique in being specific for this organ, hence abnormal concentrations of IRT in plasma or serum reflect damage to this organ. Other enzymes (amylase, lipase) have been used for many years for the diagnosis of acute pancreatitis, however, the enzymes are not organ specific and elevated levels can be caused by disease states to organs and tissues other than the pancreas. Conventional I-125 IRT radioimmunoassays employing polyclonal antibodies have been recommended as a screening test for chronic pancreatitis; however, some of these tests have not been successful because of a high interassay coefficient of variation of up to 44% (Koop H et al. Digestion, 20. 151-156, 1980). In contrast, the IRT-3B6/140-157, IRT-2D1/182, and IRT-3C4/54, IRT-4B2/41 combinations have an interassay coefficient of variation of 12-14% at 100ng/ml. These improvements now provide a reliable assay for the determination of the exocrine function of the human pancreas.

From the foregoing it has been found that the interassay co-efficient of variation may vary from 2-14% which demonstrates that the invention is particularly advantageous in regard to reproducibility compared to conventional polyclonal assay systems discussed previously.

The aforementioned advantage of introducing the label (eg. $^{125}$I, biotin or enzyme) into one of the MAb is particularly significant as IRT is particularly susceptible to denaturation if the label is coupled to the IRT antigen. This latter procedure appears to be inherently variable and for this reason is extremely difficult to reproduce and this can lead to wide variation in interassay coefficient of variation (eg. refer to the Koop et al reference discussed previously).

It will also be appreciated that coupling the label to the MAb reduces the total requirement for IRT which can only be obtained at this time from human autopsy material.

The invention may provide a positive result for CF if a sample with an IRT concentration of at least 100μg/l of body fluid was reassayed to again provide a resulting concentration greater than the 100 μg/l cut-off level. Suitably the minimum cut-off level may be between 100-150 μg/l.

Each assay may be calibrated to give a resampling rate for the general population of 1% or less.

CLAIMS:

1. A method for the preparation of monoclonal antibody (MAb) derived from immunoreactive trypsin (IRT) including the steps of:

   (i) obtaining purified human IRT or extract containing same; and

   (ii) forming antibody to said derivative or said extract by cloning antibody producing cells from an animal having been administered thereto said derivative or said extract.

2. A method as claimed in claim 1 wherein the purified IRT is obtained from human autopsy pancreas.

3. A method as claimed in claim 1 or 2 wherein the monoclonal antibody is subjected to a screening step wherein a support surface is coated with IRT antigen before being contacted with said MAb and the resulting complex subjected to a detection step to determine whether the MAb has reacted with IRT antigen.

4. A method of detection of IRT in a fluid sample including the steps of:

   (i) immunizing an animal with human purified IRT or extract containing same;

   (ii) removing a spleen from the animal;

   (iii) treating the spleen to form a cell suspension;

(iv)      purifying the cell suspension to isolate spleen white blood cells or lymphocytes;

(v)      forming hybridoma cells containing as one component said spleen white blood cells or lymphocytes;

(vi)      cloning or recloning said hybridoma cells using appropriate cell feeder layers;

(vii)      carrying out screening assays with IRT antigen so as to isolate hybridoma cells which produce MAb specific to IRT;

(viii)      contacting a fluid sample suspected of containing IRT with monoclonal antibody prepared from hybridoma cells isolated after step (vii) and

(ix)      subjecting the complex formed in step (viii) to a detection step.

5.      A method of diagnosis of cystic fibrosis (CF) including the steps of:

(i)      isolating a monoclonal antibody reactive with IRT;

(ii)      contacting said monoclonal antibody with a fluid sample from a patient suspected of suffering from CF; and

(iii)      analysing for presence of IRT in said sample to determine if the IRT reacted with said monoclonal antibody.

6. A method as claimed in claim 5 wherein biotin was bound to the monoclonal antibody in step (ii) and subsequently in step (iii) an EIA analysis was carried out wherein second monoclonal antibody labelled with peroxidase enzyme conjugated with streptavidin or avidin was reacted with the complex formed in step (ii).

7. A method as claimed in claim 5 wherein in step (ii) blood spots are incubated in biotin labelled first MAb.

8. A method as claimed in claim 7 wherein the range of the assay detection in the detection step (iii) was determined by serial dilution of standards containing varying concentrations of IRT.

9. A method as claimed in claim 8 wherein the minimum detectable plasma IRT concentration was 1.5 µg/l and this concentration increased to a maximum of 2500 µg/l.

10. A method as claimed in claim 9 wherein a positive result of CF was obtained if a sample with a IRT concentration of at least 100 µg/l of plasma was reassayed to again provide a resulting concentration greater than the 100 µg/l cut-off level.

11. A method of diagnosis of pancreatic damage including the steps of:

(i) isolating a monoclonal antibody reactive with IRT;

36.

(ii) contacting monoclonal antibody with a
fluid sample from a patient suspected of
suffering from pancreatic damage; and

(iii) analysing for presence of IRT in said
sample to determine if the IRT reacted
with the monoclonal antibody.